(19) Europäisches Patentamt
European Patent Office
Office européen des brevets

(11) Veröffentlichungsnummer: **0 043 994**
**B1**

# (12) EUROPÄISCHE PATENTSCHRIFT

(45) Veröffentlichungstag der Patentschrift: **06.06.84**

(21) Anmeldenummer: **81105132.5**

(22) Anmeldetag: **02.07.81**

(51) Int. Cl.³: **C 07 J 53/00**, C 07 J 1/00, C 07 J 17/00, C 07 J 21/00 // C07J71/00

## (54) 5Beta-Hydroxy-delta-6-steroide und Verfahren zu ihrer Herstellung.

(30) Priorität: **11.07.80 DE 3026783**

(43) Veröffentlichungstag der Anmeldung: **20.01.82 Patentblatt 82/3**

(45) Bekanntmachung des Hinweises auf die Patenterteilung: **06.06.84 Patentblatt 84/23**

(84) Benannte Vertragsstaaten: **AT BE CH DE FR GB IT LI LU NL SE**

(56) Entgegenhaltungen:
**FR - A - 2 347 383**

**HELVETIA CHIMICA ACTA, Band 62, Heft 7, 31. Oktober 1979, BASEL (CH) P. WIELAND: "Stereospezifische Synthese von 6Beta, 7Beta- Methylen - 20 - spirox-4-en-3, 21-dion (Prorenon)", Seiten 2276-2281**
**TETRAHEDRON LETTERS, Heft 20, Mai 1977, OXFORD (GB) W.G. SALMOND et al.: "Alternative modes of decomposition of allylic selenoxides diastereoisomeric at selenium. Preparation of Delta 5,7-and 5Beta-Hydroxy-Delta6-steroids", Seiten 1683-1686**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Bittler, Dieter, Bölkauer Pfad 11, D-1000 Berlin 27 (DE)**
Erfinder: **Laurent, Henry, Dr., Glambecker Weg 21, D-1000 Berlin 28 (DE)**
Erfinder: **Nickisch, Klaus, Dr., Fregestrasse 39B, D-1000 Berlin 41 (DE)**
Erfinder: **Nickolson, Robert, Dr., Fuchsienweg 12 C, D-1000 Berlin 47 (DE)**
Erfinder: **Wiechert, Rudolf, Prof. Dr., Petzowerstrasse 8A, D-1000 Berlin 39 (DE)**

**EP 0 043 994 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft den Gegenstand des Patentanspruches.

Unter Acyl sind solche Säurereste zu verstehen, die bis zu 12 C-Atome aufweisen und sich von Säuren ableiten, die in der Steroidchemie üblicherweise für Veresterungen angewandt werden. Bevorzugte Säuren sind Carbonsäuren mit 1 bis 8 Kohlenstoffatomen. Die Carbonsäuren können auch verzweigt, mehrbasisch oder in üblicher Weise, zum Beispiel durch eine Hydroxy- oder eine Aminogruppe, substituiert sein. Geeignet sind auch cycloaliphatische, aromatische, gemischt aromatischaliphatische oder heterocyclische Säuren. Als bevorzugte Säuren zur Ausbildung des Acylrestes seien beispielsweise genannt Essigsäure, Propionsäure, Capronsäure, Trimethylessigsäure, Cyclopentylpropionsäure, Cyclohexylessigsäure, Phenylpropionsäure, Phenylessigsäure, Dialkylaminoessigsäure, Piperidinoessigsäure, Bernsteinsäure und Benzoesäure.

Unter Alkyl sind solche Reste zu verstehen, die sich von aliphatischen Kohlenwasserstoffen ableiten und 1 bis 6 C-Atome aufweisen, wie z. B. Methyl, Ethyl, Propyl, Isopropyl, n-Butyl, i-Butyl und tert.-Butyl.

Die erfindungsgemäß herstellbaren Verbindungen sind Zwischenprodukte zur Herstellung von 3-Keto-$\Delta^4$-6$\beta$.7$\beta$-methylensteroiden, die pharmakologisch wertvolle Substanzen darstellen.

Es ist bekannt, daß sich 3$\beta$-Acetoxy-5-hydroxy-5$\beta$.17$\alpha$-pregn-6-en-21.17-carbolacton aus 3$\beta$-Acetoxy-5.6$\beta$-epoxy-5$\beta$-pregnan-21.17-carbolacton über die Zwischenstufe des 3$\beta$-Acetoxy-5-hydroxy-6$\alpha$-phenylseleno-5$\beta$-pregnan-17.21-carbolacton herstellen läßt (Helv. Chim. Acta 62 (1976) 2276).

Dieses Verfahren hat jedoch den Nachteil, daß man für die Synthese stark toxische Organoselen-Verbindungen, wie z. B. das Diphenyldiselenid, benötigt, deren Handhabung im technischen Maßstab Schwierigkeiten bereitet, deren Abfallbeseitigung nur über Sonderdeponien möglich ist und die als Rohstoff auch nicht in beliebigen Mengen zur Verfügung stehen dürften.

Aufgabe der vorliegenden Erfindung war es deshalb, einen selenfreien Weg zur Herstellung von 5$\beta$-Hydroxy-$\Delta^6$-steroiden zu finden.

Diese Aufgabe wurde durch das im Anspruch gekennzeichnete Verfahren gelöst.

Das erfindungsgemäße Verfahren wird so durchgeführt, daß man das 7$\alpha$-Chlor-5$\beta$.6$\beta$-epoxy-steroid in einem inerten protischen Lösungsmittel löst und mit metallischem Zink, z. B. in Pulverform, als Gries oder als Späne, in Gegenwart einer aliphatischen Carbonsäure oder verdünnter Mineralsäure zwischen Raumtemperatur und etwa 100° C rührt.

Als inerte protische Lösungsmittel sind alle solche geeignet, die nicht mit den Reaktionsmitteln reagieren. Beispielsweise genannt seien aliphatische Alkohole wie Methanol, Ethanol, n-Propanol und Isopropylalkohol, aliphatische und cycloaliphatische Ether wie Diisopropylether, Tetrahydrofuran, und Dioxan sowie Wasser.

Als aliphatische Carbonsäure ist neben der bevorzugt verwendeten Essigsäure auf Ameisensäure und Propionsäure zur Durchführung der erfindungsgemäßen Reaktion geeignet. Möglich ist aber auch die Verwendung von verdünnter Mineralsäure, wie Salzsäure oder Schwefelsäure.

Die verwendete Säure wird im Überschuß eingesetzt. Der Überschuß beträgt die 30—100fache Menge (Moläquivalente). Die Säure wird zweckmäßigerweise in einer Konzentration von 0,2—1,0 Mol/l eingesetzt.

Das Reaktionsgemisch wird erwärmt, wobei ein Temperaturbereich von 40—70° C bevorzugt ist.

Die Reaktionsdauer beträgt etwa 0,5 bis 7 Stunden, wobei diese Zeit vom verwendeten Ausgangsmaterial und insbesondere von der Temperatur abhängig ist.

Der Verlauf der erfindungsgemäßen Reaktion war insofern überraschend, da unter den angewendeten Reaktionsbedingungen (sauer, Temperatur oberhalb Raumtemperatur) zu erwarten gewesen wäre, daß sich die gebildeten 5$\beta$-Hydroxy-$\Delta^6$-steroide durch Allylumlagerung in entsprechende Folgeprodukte umwandeln würden. Aus den Arbeiten von Morand (P. Morand und A. Van Tongerloo, Steroids 21 (1973) 47—61) ist nämlich bekannt, daß sich 5-Hydroxy-$\Delta^6$-steroide schon bei Raumtemperatur in Gegenwart von 80%iger Essigsäure in die entsprechenden Allylumlagerungsprodukte, wie 7-Hydroxy- und 7-Acetoxy-$\Delta^5$-steroide, umwandeln. Darüber hinaus ist aus dieser Arbeit bekannt, daß sich beim Erwärmen von 5-Hydroxy-$\Delta^6$-steroiden mit 80%iger Essigsäure, z. T. sogar Polyene bilden.

Aus dem erfindungsgemäß hergestellten Zwischenprodukt 3$\beta$-Acetoxy-5-hydroxy-15$\beta$.16$\beta$-methylen-5$\beta$-androst-6-en-17-on läßt sich auf folgendem Wege beispielsweise das bekannte aldosteronantagonistisch wirksame 6$\beta$.7$\beta$; 15$\beta$.16$\beta$-Dimethylen-3-oxo-17$\alpha$-pregn-4-en-21.17-carbolacton herstellen:

Eine Lösung von 3$\beta$-Acetoxy-5-hydroxy-15$\beta$.16$\beta$-methylen-5$\beta$-androst-6-en-17-on in 300 ml Methanol wird mit 15 g Kaliumcarbonat 30 Minuten bei Raumtemperatur gerührt. Die Reaktionslösung wird mit Diethylether verdünnt, mit Wasser gewaschen, getrocknet und eingedampft. Es werden 27 g 3$\beta$.5-Dihydroxy-15$\beta$.16$\beta$-methylen-5$\beta$-androst-6-en-17-on erhalten. Schmelzpunkt 187—190° C (Aceton).

Eine Lösung von 26 g 3$\beta$.5-Dihydroxy-15$\beta$.16$\beta$-methylen-5$\beta$-androst-6-en-17-on in 520 ml Ethylenglykoldimethylether wird mit 78 g Zink-Kupfer und 69 ml Methylenjodid 4 Stunden bei 80° C gerührt. Anschließend wird mit Methylenchlorid verdünnt, mit gesättigter Ammoniumchloridlösung und Wasser gewaschen, getrocknet und eingedampft. Der Rückstand wird an Kieselgel chromatographiert. Es werden 16,3 g 3$\beta$.5-Dihydroxy-6$\beta$.7$\beta$; 15$\beta$.16$\beta$-dimethylen-5$\beta$-androstan-17-on vom Schmelzpunkt

205,5—207° C erhalten.

25,1 g 3β.5-Dihydroxy-6β.7β;15β.16β-dimethylen-5β-androstan-17-on werden in 500 ml Tetrahydrofuran gelöst. Unter Kühlung auf 0° C und unter Argonatmosphäre werden zu dieser Lösung 75,5 g Kaliumethylat gegeben und anschließend unter Rühren 50,4 ml Propargylalkohol in 104 ml Tetrahydrofuran gelöst zugetropft. Das Reaktionsgemisch wird 20 Stunden bei 0° C gerührt und in Eiswasser eingegossen. Nach Neutralisation mit verdünnter Schwefelsäure wird der ausgefallene Niederschlag abfiltiert und getrocknet. Das Rohprodukt wird an Kieselgel chromatographiert. Man erhält 25 g 17α-(3-Hydroxy-1-propinyl)-6β.7β;15β.16β-dimethylen-5β-androstan-3β.5.15β-triol. Schmelzpunkt 202—203° C (Aceton).

24,5 g 17α-(3-Hydroxy-1-propinyl)-6β.7β;15β.16β-dimethylen-5β-androstan-3β.5.17β-triol werden in 250 ml Tetrahydrofuran und 125 ml Methanol in Gegenwart von 3,75 g Palladium auf Kohle (10%ig) und 0,5 ml Pyridin bis zur Aufnahme von 2 Äquivalenten Wasserstoff hydriert. Es wird vom Katalysator abfiltriert und eingedampft. Man erhält 24,7 g 17α-(3-Hydroxypropyl)-6β.7β;15β.16β-dimethylen-5β-androstan-3β.5.17β-triol, das ohne weitere Aufreinigung in die nächste Stufe eingesetzt wird.

Eine Lösung von 24,7 g 17α-(3-Hydroxypropyl)-6β.7β;15β.16β-dimethylen-5β-androstan-3β.5.17β-triol in 247 ml Pyridin wird mit einer Lösung von 74,1 g Chrom-(VI)-oxid in 247 ml Wasser und 494 ml Pyridin versetzt und 16 Stunden bei 50° C gerührt. Danach wird mit Methylenchlorid verdünnt, mit Wasser gewaschen, getrocknet und eingedampft. Der Rückstand wird an Kieselgel chromatographiert. Nach Umkristallisieren aus Diisopropylether-Aceton werden 14,5 g 6β.7β;15β.16β-Dimethylen-3-oxo-17α-pregn-4-en-21.17-carbolacton vom Schmelzpunkt 196,5—197,5° C erhalten. UV: $\varepsilon_{265}$ = 18 700 (Methanol).

Geht man von 3β.5-Dihydroxy-15β.16β-methylen-5β.17α-pregn-6-en-21.17-carbolacton als erfindungsgemäß hergestelltem Zwischenprodukt aus, so erhält man z. B. auf folgendem Wege das gleiche 6β.7β;15β.16β-Dimethylen-3-oxo-17α-pregn-4-en-21.17-carbolacton:

5 g 3β.5-Dihydroxy-15β.16β-methylen-5β.17α-pregn-6-en-21.17-carbolacton werden in 100 ml Tetrahydrofuran gelöst und mit 15 g Zink-Kupfer versetzt. Dazu tropft man innerhalb von 7 Stunden 13,2 ml Methylenjodid so zu, daß die Temperatur 30° C nicht übersteigt und rührt weitere 10 Stunden bei Raumtemperatur. Zur Entfernung des Metalls wird über Celite filtiert, das Filtrat mit Methylenchlorid verdünnt und mit gesättigter Ammonchloridlösung gewaschen, über Magnesiumsulfat getrocknet und im Vakuum eingeengt. Der Rückstand wird an Kieselgel chromatographiert. Man erhält 4,4 g 3β.5-Dihydroxy-6β.7β;15β.16β-dimethylen-5β.17α-pregnan-21.17-carbolacton als Öl.

2,8 g 3β.5-Dihydroxy-6β.7β;15β.16β-dimethylen-5β.17α-pregnan-21.17-carbolacton werden in 28 ml Pyridin gelöst und mit einer Lösung von 15 g Chrom(VI)-oxid in 28 ml Pyridin und 14 ml Wasser versetzt und 16 Stunden bei 50° C gerührt. Nach dem Abkühlen wird mit Methylenchlorid verdünnt, mit Wasser gewaschen, über Magnesiumsulfat getrocknet und im Vakuum eingeengt. Der Rückstand wird an Kieselgel chromatographiert. Man erhält 2,3 g 6β.7β;15β.16β-Dimethylen-3-oxo-17α-pregn-4-en-21.17-carbolacton vom Schmelzpunkt 198—198,5° C.

Die nachfolgenden Beispiele sollen das erfindungsgemäße Verfahren erläutern.

## Beispiel 1

Eine Lösung von 21 g 3β-Acetoxy-7α-chlor-5β.6β-epoxy-17.17.-ethylendioxy-15β.16β-methylen-5β-androstan in 105 ml Tetrahydrofuran, 105 ml Essigsäure und 27 ml Wasser wird mit 63 g Zinkstaub versetzt und 1,5 Stunden bei 80° C gerührt. Es wird dann vom Zink abdekantiert, mit Methylenchlorid nachgewaschen und die organische Phase mit Natriumhydrogencarbonatlösung und Wasser gewaschen. Nach dem Trocknen und Eindampfen wird der Rückstand an Kieselgel chromatographiert. Es werden 13,8 g 3β-Acetoxy-5-hydroxy-15β.16β-methylen-5β-androst-6-en-17-on vom Schmelzpunkt 191—194° C erhalten.

### Das Ausgangsmaterial wurde wie folgt hergestellt

A. Eine Lösung von 95 g 3β-Hydroxy-15β.16β-methylen-5-androstan-17-on in 190 ml Pyridin wurde mit 95 ml Acetanhydrid versetzt und 1,5 Stunden bei 95° C gerührt. Der nach Eiswasserfällung erhaltene Niederschlag wurde abfiltriert, gut mit Wasser gewaschen und getrocknet. Es wurden 107 g 3β-Acetoxy-15β.16β-methylen-5-androsten-17-on als Rohprodukt erhalten. Eine mit Diisopropylether ausgekochte Probe schmolz bei 140,5—141° C.

B. Eine Lösung von 105 g 3β-Acetoxy-15β.16β-methylen-5-androsten-17-on in 1,05 ml Methylenchlorid wurde mit 315 ml Ethylenglykol, 210 ml o-Ameisensäuretriethylester und 10,5 g p-Toluolsulfonsäure versetzt und 1 Stunde bei Raumtemperatur gerührt. Die Reaktionslösung wurde mit 40 ml Pyridin versetzt und mit Ether verdünnt, anschließend mit Wasser gewaschen, getrocknet und eingedampft. Der Rückstand wurde mit Pentan verrieben und abgesaugt. Es wurden 109 mg 3β-Acetoxy-17.17-ethylen-dioxy-15β.16β-methylen-5-androsten vom Schmelzpunkt 177—178,5° C erhalten.

C. Zu 600 ml auf —20° C abgekühltes Methylenchlorid wurden nacheinander unter Rühren 81 g über

Phosphorpentoxid getrocknetes Chrom(VI)-oxid und 84 g 3.5-Dimethylpyrazol gegeben. Das Gemisch wurde 30 Minuten gerührt, mit 23,2 g 3β-Acetoxy-17.17-ethylendioxy-15β.16β-methylen-5-androstan versetzt und 4 Stunden bei −10°C nachgerührt. Anschließend wurde die Reaktionslösung mit 342 ml 5N Natronlauge versetzt, 1 Stunde bei 0°C gerührt, mit 120 ml Diethylether versetzt und weitere 30 Minuten gerührt. Die wäßrige Phase wurde abgetrennt und die organische Phase mit Wasser gewaschen, getrocknet und eingedampft. Der Rückstand wurde an Kieselgel chromatographiert. Es wurden 17,2 g 3β-Acetoxy-17.17-ethylendioxy-15β.16β-methylen-5-androsten-7-on vom Schmelzpunkt 193—197°C erhalten.

D. 31 g 3β-Acetoxy-17.17-ethylendioxy-15β.16β-methylen-5-androsten-7-on wurden in 310 ml Tetrahydrofuran mit 31 g Lithiumtri-tert.-butocyalanat bei Raumtemperatur innerhalb von 1,5 Stunden umgesetzt. Die Reaktionslösung wurde mit Diehtylether verdünnt, mit Kaliumnatriumtartratlösung und Wasser gewaschen, getrocknet und eingedampft. Der Rückstand wurde an Kieselgel chromatographiert. Man erhielt 24,3 g 3β-Acetoxy-17.17-ethylendioxy-15β.16β-methylen-5-androsten-7β-ol vom Schmelzpunkt 199,5—200°C (Aceton).

E. Eine Lösung von 23,3 g 3β-Acetoxy-17.17-ethylendioxy-15β.16β.-methylen-5-androsten-7β-ol in 350 ml Toluol wurde bei 80°C, nach Zugabe von Vanadium(IV)-oxidacetylacetonat, innerhalb von 35 Minuten mit 23,3 ml 80%igem tert.-Butylhydroperoxid, gelöst in 115 ml Toluol, tropfenweise versetzt. Die Reaktionslösung wurde weitere 30 Minuten bei 80°C gehalten, anschließend abgekühlt, mit Diethylether verdünnt, mit Natriumhydrogensulfitlösung, Natriumhydrogencarbonatlösung und Wasser gewaschen, getrocknet und eingedampft. Man erhielt 24,5 g 3β-Acetoxy-5.6β-epoxy-17.17-ethylendioxy-15β.16β-methylen-5β-androstan-7β-ol vom Schmelzpunkt 162—163°C (Diisopropylether).

F. Eine Lösung von 24 g 3β-Acetoxy-5.6β-epoxy-17.17-ethylendioxy-15β.16β-methylen-5β-androstan-7β-ol in 48 ml Pyridin und 48 ml Tetrachlorkohlenstoff wurde mit 28 g Triphenylphosphin versetzt und 1,5 Stunden bei Raumtemperatur gerührt. Das Reaktionsgemisch wurde mit Methylenchlorid verdünnt, mit Wasser gewaschen, getrocknet, und eingedampft. Der Rückstand wurde an Kieselgel chromatographiert. Es wurden 21,5 g 3β-Acetoxy-7α-chlor-5.6β-epoxy-17.17-ethylendioxy-15β.16β-methylen-5β-androstan erhalten vom Schmelzpunkt 169—170°C (Diisopropylether).

## Beispiel 2

200 mg 17β-Acetoxy-3β-benzoyloxy-7α-chlor-5.6β-epoxy-15β.16β-methylen-5β-androstan werden in 4 ml Essigsäure und 4 ml Propan-2-ol mit 800 mg Zinkstaub 1 Stunde bei 80°C gerührt. Es wird vom Zink abfiltriert, mit Diethylether nachgewaschen und analog Beispiel 1 aufgearbeitet. Nach Chromatographie und Umkristallisieren aus Diisopropylether-Aceton werden 135 mg 17β-Acetoxy-3β-benzoyloxy-15β.16β-methylen-5β-androst-6-en-5-ol vom Schmelzpunkt 212—212,5°C erhalten.

### Herstellung des Ausgangsmaterials

A. Eine Lösung von 5,0 g 3β-Hydroxy-15β.16β-methylen-5-androsten-17-on in 50 ml Pyridin wurde unter Eiskühlung mit 5 ml Benzoylchlorid versetzt und anschließend 17 Stunden bei Raumtemperatur gerührt. Nach Zugabe von 10 ml Wasser wurde eine weitere Stunde gerührt, dann die Reaktionslösung mit Methylenchlorid verdünnt und mit Natriumcarbonatlösung und Wasser gewaschen. Nach dem Trocknen und Eindampfen wurde der Rückstand mit Diisopropylether verrieben und abgesaugt. Es wurden 6,4 g 3β-Benzoyloxy-15β.16β-methylen-5-androsten-17-on vom Schmelzpunkt 250—258°C erhalten.

B. 6,4 g 3β-Benzoyloxy-15β.16β-methylen-5-androsten-17-on wurden in 64 ml Tetrahydrofuran mit 6,4 g Lithium-tri-tert.-butoxyalanat eine Stunde bei Raumtemperatur gerührt. Danach wurde die Lösung mit Ether verdünnt, mit 2N Schwefelsäure und Wasser gewaschen, getrocknet, und eingedampft. Als Rückstand wurden 6,5 g 3β-Benzoyloxy-15β.16β-methylen-5-androsten-17β-ol erhalten, das ohne weitere Aufreinigung in die nächste Stufe eingesetzt wurde.

C. 6,5 g 3β-Benzoyloxy-15β.16β-methylen-5-androsten-17β-ol, 13 ml Acetanhydrid und 26 ml Pyridin wurden 1,5 Stunden auf 95°C erhitzt. Nach Eiswasserfällung wurde der Niederschlag abfiltriert und in Methylenchlorid aufgenommen. Die Lösung wurde mit Wasser gewaschen, getrocknet und eingedampft. Man erhielt 6,9 g rohes 17β-Acetoxy-3β-benzoyloxy-15β.16β-methylen-5-androsten, das ohne weitere Aufreinigung in die nächste Stufe eingesetzt wurde.

D. 6,9 g 17β-Acetoxy-3β-benzoyloxy-15β.16β-methylen-5-androsten wurden in 69 ml Tetrachlorkohlenstoff mit einer essigsauren tert.-Butylchromatlösung, die aus 10,35 g Chrom(VI)-oxid, 90 ml Tetrachlorkohlenstoff, 28,2 ml tert.-Butylalkohol, 37,2 ml Essigsäure und 134 ml Acetanhydrid hergestellt worden war, versetzt. Die Reaktionslösung wurde anschließend 32 Stunden bei 80°C gerührt, mit Methylenchlorid verdünnt und mit Natriumacetatlösung, Natriumhydrogencarbonatlösung und Wasser gewaschen. Nach dem Trocknen und Eindampfen wurde der erhaltene Rückstand an Kieselgel chromatographiert. Man erhielt 4,4 g 17β-Acetoxy-3β-benzoyloxy-15β.16β-methylen-5-androsten-7-on vom Schmelzpunkt 245—246°C (Aceton-Diisopropylether).

E. Das so erhaltene 7-Keton wurde analog Beispiel 1, wie unter Herstellung des Ausgangsmaterials bei den Stufen D—F beschrieben, mit Lithiumtri-tert.-butoxyalanat zum 17β-Acetoxy-3β-benzoyloxy-15β.16β-methylen-5-androsten-7β-ol reduziert, mit tert.-Butylhydroperoxid in Gegenwart von Vanadin-(IV)-ocidacetylacetonat zu 17β-Acetoxy-3β-benzoyloxy-5.6β-epoxy-15β.16β-methylen-5β-androstan-7β-ol epoxidiert und anschließend mit Triphenylphosphin in Tetrachlorkohlenstoff und Pyridin zum 17β-Acetoxy-3β-benzoyloxy-7α-chlor-5.6β-epoxy-15β.16β-methylen-5β-androstan, Schmelzpunkt 201,5—206° C (Diisopropylether-Aceton), umgesetzt.

## Beispiel 3

13,5 g 3β.17β-Dibenzoyloxy-7α-chlor-5.6β-epoxy-15β.16β-methylen-5β-androstan werden in 130 ml Essigsäure und 130 ml Propan-2-ol mit 49,5 g Zinkstaub analog Beispiel 2 umgesetzt und aufgearbeitet. Nach Chromatographie an Kieselgel werden 8,8 g 3β.17β-Dibenzoyloxy-15β.16β-methylen-5β-androst-6-en-5-ol erhalten. Schmelzpunkt 223—225° C (Aceton-Diisopropylether).

### Herstellung des Ausgangsmaterials

A. Eine Lösung von 21 g 3β-Hydroxy-15β.16β-methylen-5-androsten-17-on in 210 ml Tetrahydrofuran wurde mit 21 g Lithiumtri-tert.-butoxyalanat 1 Stunde bei Raumtemperatur gerührt. Danach wurde mit Diethylether verdünnt, mit 2N Schwefelsäure und Wasser gewaschen, getrocknet und eingedampft. Es wurden 19 g 15β.16β-Methylen-5-androsten-3β.17β-diol erhalten, die ohne weitere Aufreinigung in die nächste Stufe eingesetzt wurden.

B. Das 15β.16β-Methylen-5-androsten-3β.17β-diol wurde analog Beispiel 2, wie unter Herstellung des Ausgangsmaterials beschrieben, mit Benzoylchlorid zu 3β.17β-Dibenzoyloxy-15β.16β-methylen-5-androsten umgesetzt, mit tert.-Butylchromat zum 3β.17β-Dibenzoyloxy-15β.16β-methylen-5-androsten-7-on oxidiert und mit Lithiumtri-tert.-butoxyalanat zum 3β.17β-Dibenzoyloxy-15β.16β-methylen-5-androsten-7β-ol reduziert, das mit tert.-Butylhydroperoxid zum 3β.17β-Dibenzoyloxy-5.6β-epoxy-15β.16β-methylen-5β-androsten umgesetzt und mit Triphenylphosphin in Tetrachlorkohlenstoff und Pyridin in das 3β.17β-Dibenzoyloxy-7α-chlor-5.6β-epoxy-15β.16β-methylen-5β-androstan überführt wurde. Das nach Chromatographie und Umkristallisieren aus Diisopropylether-Aceton erhaltene Produkt schmolz bei 197,5—198,5° C.

## Beispiel 4

3,0 g 17β-Acetoxy-3β-benzoyloxy-7α-chlor-5.6β-epoxy-5β-androstan werden in 30 ml Essigsäure und 30 ml Propan-2-ol mit 9 g Zinkstaub analog Beispiel 2 umgesetzt und aufgearbeitet. Es werden 2,8 g 17β-Acetoxy-3β-benzoyloxy-5β-androst-6-en-5-ol erhalten. Schmelzpunkt 180—185° C.

### Herstellung des Ausgangsmaterials

3β-Hydroxy-5-androsten-17-on wurde analog Beispiel 2A—C in das 17β-Acetoxy-3β-benzoyloxy-5-androsten überführt. Analog Beispiel 2D wurde daraus das 17β-Acetoxy-3β-benzoyloxy-5-androsten-7-on mit tert.-Butylchromat hergestellt. Analog Beispiel 1D—F wurde reduziert, epoxidiert und chloriert, wobei 17β-Acetoxy-3β-benzoyloxy-7α-chlor-5.6β-epoxy-5β-androstan erhalten wurde, das nach Chromatographie an Kieselgel bei 173—175,5° C schmolz.

## Beispiel 5

300 mg 3β-Acetoxy-7α-chlor-5.6β-epoxy-15β.16β-methylen-5β.17α-pregnan-21.17-carbolacton werden in 15 ml Propan-2-ol und 15 ml Essigsäure mit 900 mg Zinkstaub 2 Stunden bei 80° C gerührt. Nach der Filtration wird mit Methylenchlorid verdünnt und mit Natriumhydrogencarbonatlösung und Wasser gewaschen, über Magnesiumsulfat getrocknet und im Vakuum eingedampft. Der Rückstand wird durch präparative Dünnschichtchromatographie gereinigt. Man erhält 150 mg 3β-Acetoxy-5-hydroxy-5β.17α-pregn-6-en-21.17-carbolacton vom Schmelzpunkt 209—211° C.

### Herstellung des Ausgangsmaterials

A. Eine Suspension von 20 g 3β-Hydroxy-15β.16β-methylen-5-androsten-17-on in 400 ml Benzol wurde durch Abdestillieren von 40 ml Flüssigkeit azeotrop getrocknet und bei Raumtemperatur mit 50 ml

destilliertem Dihydropyran und 250 ml Toluolsulfonsäure versetzt. Nach 1 Stunde bei Raumtemperatur wurden 4 ml Pyridin zugegeben, mit Diethylether verdünnt, mit Natriumhydrogencarbonatlösung und Wasser gewaschen und über Magnesiumsulfat getrocknet. Das nach dem Eindampfen erhaltene Rohprodukt wurde mit Methanol ausgekocht, filtriert und getrocknet. Man erhielt 22,4 g 15β.16β-Methylen-3β-(tetrahydropyran-2-yloxy)-5-androsten-17-on vom Schmelzpunkt 175—178° C.

B. Es wurden 22 g 15β.16β-Methylen-3β-(tetrahydropyran-2-yloxy)-5-androsten-17-on in 400 ml Tetrahydrofuran gelöst, unter Eiskühlung und unter Argonatatmosphäre 65 g Kaliumethylat hinzugegeben und in diese Suspension unter Rühren eine Lösung von 44 ml Propargylalkohol in 88 ml Tetrahydrofuran getropft. Danach wurde 4 Stunden bei Raumtemperatur unter Argon gerührt. Diese Lösung wurde in 5 Liter gesättigte Kochsalzlösung gegossen und mit Essigsäure neutralisiert. Der Niederschlag wurde abfiltriert und in Methylenchlorid aufgenommen. Die Lösung wurde gewaschen, getrocknet und eingedampft. Das erhaltene Rohprodukt wurde mit Essigester verrieben. Man erhielt 23 g 17α-(3-Hydroxy-1-propinyl)-15β.16β-methylen-3β-(tetrahydropyran-2-yloxy)-5-androsten-17β-ol vom Schmelzpunkt 193—196° C.

C. Eine Lösung von 22 g 17α-(3-Hydroxy-1-propinyl)-15β.16β-methylen-3β-(tetrahydropyran-2-yloxy)-5-androsten-17β-ol in 1,3 l Methanol wurde mit 3 Löffel Raney-Nickel versetzt und unter einer Wasserstoffatmosphäre geschüttelt, wobei 2,27 l Wasserstoff aufgenommen wurden. Danach wurde der Katalysator abgesaugt, das Filtrat im Vakuum eingedampft und der Rückstand aus Essigester umkristallisiert. Man erhielt 16,75 g 17α-(3-Hydroxypropyl)-15β.16β-methylen-3β-(tetrahydropyran-2-yloxy)-5-androsten-17β-ol vom Schmelzpunkt 156—160° C.

D. Zu einer Lösung von 15 g 17α-(3-Hydroxypropyl)-15β.16β-methylen-3β-(tetrahydropyran-2-yloxy)-5-androsten-17β-ol in 1,2 l Dimethylformamid wurden 48 g Pyridiniumdichromat gegeben und 3 Stunden bei Raumtemperatur gerührt. Danach wurde in gesättigte Kochsalzlösung eingegossen, der Niederschlag abfiltriert und mit Methanol ausgewaschen. Das erhaltene Rohprodukt wurde aus Methanol umkristallisiert. Man erhielt 14,3 g 3β-(Tetrahydropyran-2-yloxy)-15β.16β-methylen-17α-pregn-5-en-21.17-carbolacton vom Schmelzpunkt 218—220° C.

E. 14,3 g 3β-(tetrahydropyran-2-yloxy)-15β.16β-methylen-17α-pregn-5-en-21.17-carbolacton wurden in 250 ml Methanol und 150 ml Methylenchlorid gelöst, 2 ml 3N Salzsäure hinzugegeben und 2 Stunden bei Raumtemperatur gerührt. Danach wurde im Vakuum eingedampft, der Rückstand in Methylenchlorid gelöst und neutralgewaschen. Das nach dem Eindampfen angefallene Rohprodukt wurde mit Methanol ausgekocht. Man erhielt 11,6 g 3β-Hydroxy-15β.16β-methylen-17α-pregn-5-en-21.17-carbolacton vom Schmelzpunkt 216—223° C.

F. 750 mg 3β-Hydroxy-15β.16β-methylen-17α-pregn-5-en-21.17-carbolacton wurden in 10 ml Pyridin gelöst, mit 10 ml Acetanhydrid versetzt und eine Stunde auf dem Dampfbad erhitzt. Danach wurde in Eiswasser eingegeben, der Niederschlag abfiltriert und im Vakuum getrocknet. Das so erhaltene Rohprodukt wurde durch Gradientenchromatographie gereinigt. Man erhielt 683 mg 3β-Acetoxy-15β.16β-methylen-17α-pregn-5-en-21.17-carbolacton vom Schmelzpunkt 224—226° C.

G. Das so erhaltene 3β-Acetoxy-15β.16β-methylen-17α-pregn-5-en-21.17-carbolacton wurde analog Beispiel 1C—F in das 3β-Acetoxy-7α-chlor-5.6β-epoxy-15β.16β-methylen-5β.7α-pregnan-21.17-carbolacton überführt.

Beispiel 6

61 g 3β-(Tetrahydropyran-2-yloxy)-7α-chlor-5.6β-epoxy-15β.16β-methylen-5β.17α-pregnan-21.17-carbolacton werden in 400 ml Tetrahydrofuran, 400 ml Essigsäure und 160 ml Wasser suspendiert. Die Suspension wird innerhalb von 45 Minuten mit 150 g Zinkstaub in 3 Portionen versetzt und 4,5 Stunden auf 70° C erhitzt. Nach dem Abkühlen wird in Eiswasser eingerührt, mit Natriumhydrogencarbonat neutralisiert und Methylenchlorid extrahiert. Der Extrakt wird nach dem Waschen mit Wasser über Magnesiumsulfat getrocknet und im Vakuum eingedampft. Der Rückstand wird an Kieselgel chromatographiert. Man erhielt 37,9 g 3β.5-Dihydroxy-15β.16β-methylen-5β.17α-pregn-6-en-21.17-carbolacton vom Schmelzpunkt 237—238° C.

Herstellung des Ausgangsmaterials

3β-(Tetrahydropyran-2-yloxy)-15β.16β-methylen-17α-pregn-5-en-21.17-carbolacton wurde analog Beispiel 1 C—F in das 3β-(Tetrahydropyran-2-yloxy)-7α-chlor-5.6β-epoxy-15β.16β-methylen-5β.17α-pregnan-21.17-carbolacton überführt.

**Patentansprüche**

Verfahren zur Herstellung von 5β-Hydroxy$\Delta^6$-steroiden der allgemeinen Formel

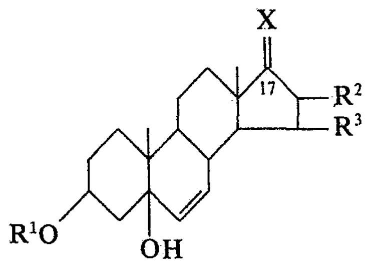

worin
R¹ Wasserstoff, Acyl, niederes Alkyl oder den Tetrahydropyranylrest und
R², R³ einzeln jeweils Wasserstoff oder gemeinsam Methylen darstellen und
X für Sauerstoff, die Gruppierung

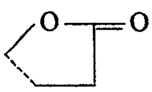 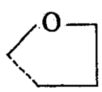 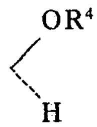 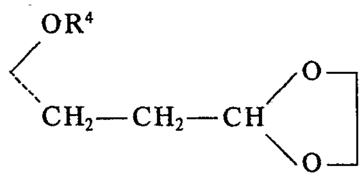 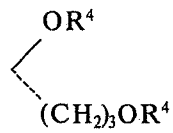

(mit $R^4$ in der Bedeutung von Wasserstoff oder Acyl) und

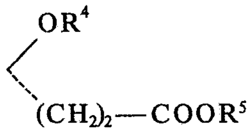

(mit $R^5$ in der Bedeutung von Wasserstoff oder niederem Alkyl) steht, dadurch gekennzeichnet, daß man auf entsprechende 7α-Chlor-5β.6β-epoxy-steroide in einem inerten Lösungsmittel niedere aliphatische Carbonsäuren oder verdünnte Mineralsäuren in Gegenwart von metallischem Zink bei Temperaturen zwischen Raumtemperatur und 100°C, vorzugsweise bei 40—70°C, einwirken läßt.

**Claim**

Process for the manufacture of 5β-hydroxy-$\Delta^6$-steroids of the general formula

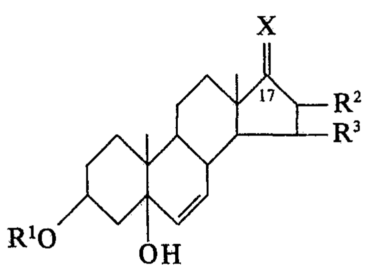

in which
R¹ represents hydrogen, acyl, lower alkyl or a tetrahydropyranyl radical,
R², R³ each represents hydrogen or together represent methylene, and
X represents oxygen or the grouping

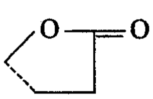 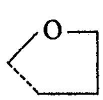 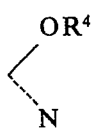 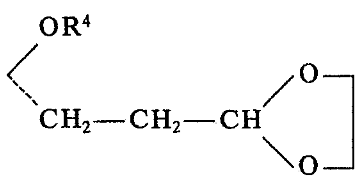 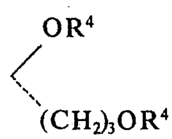

(in which $R^4$ represents hydrogen or acyl), and

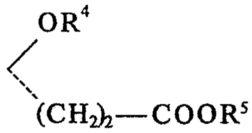

(in which $R^5$ represents hydrogen or lower alkyl), characterized in that lower aliphatic carboxylic acids or dilute mineral acids in the presence of metallic zinc are allowed to act on corresponding 7α-chloro-5β,6β-epoxysteroids in an inert solvent at temperatures of between room temperature and 100° C, preferably at from 40 to 70° C.

**Revendication**

Procédé de préparation de 5β-hydroxy $\Delta^6$-stéroïdes répondant à la formule générale:

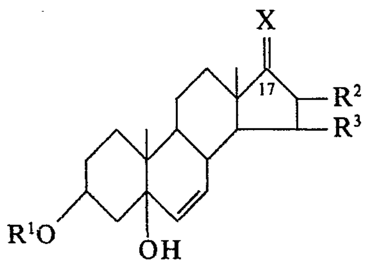

dans laquelle
$R^1$ représente l'hydrogène, un radical acyle, un radical alkyle inférieur ou le radical tétrahydropyrannyle,
$R^2$ et $R^3$ représentent chacun l'hydrogène ou forment ensemble un radical méthylène et
X représente l'oxygène, l'un des radicaux:

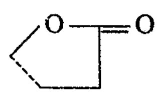 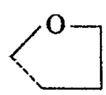 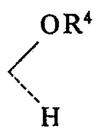 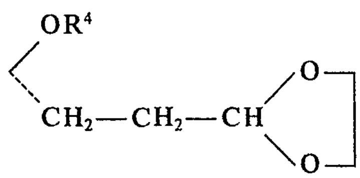 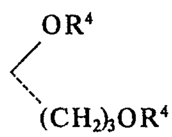

(où $R^4$ représente l'hydrogène ou un acyle) ou un radical:

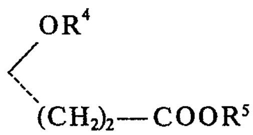

(où $R^5$ représente l'hydrogène ou un radical alkyle inférieur), procédé caractérisé en ce qu'on fait agir sur des chloro-7α époxy-5β,6β stéroïdes correspondants, dans un solvant inerte, des acides carboxyliques aliphatiques inférieurs ou des acides minéraux dilués, en présence de zinc métallique, à des températures comprises entre la température ambiante et 100° C, de préférence entre 40 et 70° C.